Europäisches Patentamt

European Patent Office

Office européen des brevets

(19)

(11) Veröffentlichungsnummer: **0 035 211**
B1

(12) **EUROPÄISCHE PATENTSCHRIFT**

(45) Veröffentlichungstag der Patentschrift: **28.12.83**

(51) Int. Cl.³: **G 01 N 33/92, C 12 Q 1/44**

(21) Anmeldenummer: **81101302.8**

(22) Anmeldetag: **23.02.81**

(54) **Verfahren und Reagens zur Bestimmung der beta-Lipoproteine (LDL).**

(30) Priorität: **29.02.80 DE 3007764**

(43) Veröffentlichungstag der Anmeldung:
**09.09.81 Patentblatt 81/36**

(45) Bekanntmachung des Hinweises auf die Patenterteilung:
**28.12.83 Patentblatt 83/52**

(84) Benannte Vertragsstaaten:
**AT BE CH DE FR GB IT LI LU NL SE**

(56) Entgegenhaltungen:
**EP - A - 0 013 814**
**DE - A - 2 013 644**
**DE - A - 2 708 912**
**US - A - 4 039 285**

**CLINICAL CHEMISTRY, Band 25, Nr. 4, April 1979, Seiten 596-604 Easton, Pennsylvania, U.S.A. G.R. WARNICK et al.: "Comparison of current methods for high-density lipoprotein cholesterol quantitation"**

**CHEMICAL ABSTRACTS, Band 91, Nr. 11, 10. September 1979, Seite 378, Nr. 86842q Columbus, Ohio, U.S.A. M.L. GIRARD:**

(73) Patentinhaber: **BOEHRINGER MANNHEIM GMBH Patentabteilung, Abt. E Sandhofer Strasse 112-132 Postfach 31 01 20**
**D-6800 Mannheim 31 Waldhof (DE)**

(72) Erfinder: **Ziegenhorn, Joachim, Dr.**
**Ina-Seidel-Weg 1**
**D-8130 Starnberg (DE)**
Erfinder: **Bartl, Knut, Dr.**
**Am Westend 6**
**D-8121 Wilzhofen (DE)**
Erfinder: **Brandhuber, Max**
**Ammerstrasse 8**
**D-8120 Weilheim (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22**
**D-8000 München 86 (DE)**

(56) Entgegenhaltungen:
**"Precipitation of low-density serum lipoproteins by polyanions"**
**CHEMICAL ABSTRACTS, Band 87, Nr. 3, 18. Juli 1977, Seite 268, Nr. 18535j Columbus, Ohio, U.S.A. M.F. LOPES-VIRELLA et al.: "Cholesterol determination in high-density lipoproteins separated by three different methods"**

# 0035211

## Verfahren und Reagens zur Bestimmung der $\beta$-Lipoproteine (LDL)

Die Erfindung betrifft ein Verfahren und ein Reagens zur Bestimmung der $\beta$-Lipoproteinfraktion (Low Density Lipoprotein (LDL)) in Körperflüssigkeiten.

Hypercholesterinämie und Hypertriglyceridämie begünstigen die Entstehung der Atherosklerose und des Herzinfarkts. Die Bestimmung von Cholesterin und Triglyceriden im Serum gehören daher zu den am häufigsten durchgeführten Tests im klinisch-chemischen Routinelabor.

Zahlreiche Untersuchungen zum Fettstoffwechsel kommen zu dem Schluß, daß das individuelle Coronarrisiko besser erfaßt werden kann, wenn nicht nur die Veränderung im Triglycerid- und Cholesterinspiegel bestimmt, sondern die zugrundeliegenden pathologischen Verschiebungen im Lipoptroeinmuster ermittelt werden (Münch. med. Wschr. *121* (1979) 1639).

Die bekannten Plasmalipoproteine enthalten einen unterschiedlich hohen Anteil an Protein, Phospholipiden, Cholesterin und Triglyceriden. Sie lassen sich aufgrund ihres Verhaltens (unterschiedliche Dichte) in der analytischen Ultrazentrifuge in drei verschiedene Klassen unterteilen:

Prä-$\beta$-Lipoprotein $=$ VLDL (Very Low Density Lipoprotein)
$\beta$-Lipoprotein $\quad=$ LDL (Low Density Lipoprotein)
$\alpha$-Lipoprotein $\quad=$ HDL (High Density Lipoprotein).

Die Untersuchung der Funktion der Lipoproteine ergab, daß LDL innerhalb der Lipoproteine die entscheidende atherogene Komponente darstellt, bei deren Erhöhung im Blut ein gesteigertes Risiko für eine coronare Herzkrankheit vorliegt. Die frühzeitige Erkennung und Bekämpfung dieses Zustands ist von großer Bedeutung. Es besteht daher ein Bedarf nach einem praktikablen Verfahren zur quantitativen Bestimmung der LDL-Konzentration im Serum und Plasma.

Bisher wurden für die Bestimmung der LDL-Lipoproteinfraktion im wesentlichen drei Methoden eingesetzt, die jedoch Nachteile besitzen:

1. Ultrazentrifugation

Dieses Verfahren ist für ein Routinelabor nicht geeignet, da man hierzu eine spezielle Geräteausrüstung benötigt und die Durchführung eine äußerst sorgfältige Arbeitstechnik und einen sehr hohen Zeitaufwand (2 x 30 Stunden bei 105 000 g) erfordert. Somit blieb dieses Analysenverfahren bisher nur dem Labor der forschenden Medizin vorbehalten.

2. Fällunsreaktion

Der LDL-Gehalt kann durch fraktionierte Fällung mit einem Polyanion, wie Heparin-Na oder Dextransulfat, und einem zweiwertigen Kation, wie $Ca^{++}$, $Mn^{++}$, $Mg^{++}$, ebenfalls bestimmt werden. Die Lipoproteine lassen sich nämlich mit steigender Konzentration des Polyanions in der folgenden Reihenfolge ausfällen: VLDL, LDL und HDL. Dieses Verfahren erfordert jedoch zwei Arbeitsschritte und ist somit unpraktikabel und nicht automatisierbar: VLDL wird in einem ersten Fällungsschritt abgetrennt, anschließend wird durch Erhöhung der Konzentration des Fällungsmittels die LDL-Lipoproteinfraktion gefällt und turbidimetrisch bestimmt (H. Okabe, X. Int. Congr. of Clin. Chem., Mexico (1978)).

3. Bestimmung der LDL-Konzentration über die Friedewald-Formel

Bei diesem Verfahren bestimmt man den Triglycerid-, Cholesterin- und HDL-Cholesteringehalt der Probe und berechnet daraus den Gehalt an LDL-Cholesterin nach dem Verfahren von Friedewald, Clin. Chem. *18* (1972) 499. Dieses umständliche Verfahren ist ebenfalls unpraktikabel.

4. Qualifizierung nach elektrophoretischer Trennung und Polyanionenfällung

Dieses Verfahren jedoch ist zeitaufwendig und erfordert eine eigene Elektrophorese-Apparatur sowie ein Densitometer zur Auswertung (Lab. Med. *1* (1977) 145).

Der Erfindung lag daher die Aufgabe zugrunde, ein praktikables, automatisierbares Verfahren zu schaffen, mit dem LDL im Routinelabor unmittelbar bestimmt werden kann. Gelöst wird diese Aufgabe erfindungsgemäß durch ein Verfahren zur direkten turbidimetrischen Bestimmung der $\beta$-Lipoproteine (LDL) in Körperflüssigkeiten, wie Serum oder Plasma durch Fällung mit einem Polyanion und einem zweiwertigen Kation aus wäßriger Lösung, welches dadurch gekennzeichnet ist, daß man die Fällung in Gegenwart von 1 bis 35 g pro liter eines mehrzahligen Liganden als Komplexbildner bei einem pH-Wert zwischen 7 und 9 durchführt.

Das erfindungsgemäße Verfahren beruht auf der Auffindung der überraschenden Tatsache, daß LDL in Gegenwart von VLDL und HDL selektiv und praktisch quantitativ gefällt und turbidimetrisch bestimmt werden kann, wenn man die Fällung mit der Kombination von Komplexierungsmittel, Polyanion und Kation durchführt. Dies ist überraschend, da nach den bisher publizierten Untersuchungen bei Ausfällung von LDL stets VLDL mitfällt, wenn es nicht vorher abgetrennt wurde (M. Burstein, H.R. Scholnick in "Protides of the Biological Fluids", Ed. Peeters, S. 21—28 (1972); Ärztl. Lab. *23*, 101—110 (1977)). Erfindungsgemäß gelingt es dagegen überraschenderweise, LDL so selektiv zu fällen, daß sogar eine kinetische Messung, also Messung innerhalb eines vorbestimmten Zeitintervalls, durchführbar ist.

Gleichzeitig wird der Fehler gegenüber dem bekannten Verfahren der gemeinsamen Fällung von VLDL und LDL wesentlich verringert.

Als Komplexbildner werden in Rahmen der Erfindung Verbindungen verstanden, die zur Bildung von Komplexen befähigt sind, also von Verbindungen höherer Ordnung, die durch Zusammenschluß von Molekülen entstehen. Als speziell brauchbar erwiesen sich die vielzähligen Liganden, wie dreizählige, vierzählige und sechszählige Liganden. Bevorzugt werden die sechszähligen Liganden, wie die Aminopolycarbonsäuren, beispielsweise Äthylendiamintetraessigsäure (EDTA), Nitrilotriessigsäure (NTA) und Diäthylentriaminpentaessigsäure.

Die Komplexbildnermenge liegt vorzugsweise zwischen 5 und 25 g/l.

Als Kation werden im Rahmen der Erfindung Salze zweiwertiger Kationen mit einem die Reaktion nicht störenden Anion verwendet. Bevorzugt werden zweiwertige Kationen aus der Gruppe der Erdalkalimetalle verwendet. Als Beispiele für andere geeignete zweiwertige Kationen seien Mangan und Kobalt genannt. Besonders bevorzugt wird Calcium.

Gemäß einer bevorzugten Ausführungsform der Erfindung werden Kation und Komplexbildner zuerst miteinander umgesetzt unter Bildung eines salzartigen Komplexes, welcher gegebenenfalls nach vorheriger Isolierung für das erfindungsgemäße Verfahren eingesetzt wird. Wenn der Komplex aus der Lösung, in der er gebildet wird, zuerst abgetrennt wird, kommt es zwar nicht auf die Verwendung der Komponenten in stöchiometrischer Menge an, da das jeweils überschüssige und nicht an der Komplexbildung teilnehmende Mittel mit dem Lösungsmittel abgetrennt wird. Vorzugsweise wird man jedoch Kation und Komplexbildner in stöchiometrischer Menge einsetzen. Dies gilt auch dann, wenn die Komplexbildung direkt in der zu untersuchenden Lösung vorgenommen wird. Da es häufig zweckmäßig ist, einen Überschuß an Kation bei der Fällung zugegen zu haben, setzt man bei dieser Ausführungsform zweckmäßig Komplexbildner und Kation zuerst in stöchiometrischer Menge zu und bringt das überschüssige Kation erst nach der Komplexbildung ein. Die Konzentration an Kation in der Lösung liegt zweckmäßig zwischen 0,01 und 0,3 M, zweckmäßiger zwischen 0,08 und 0,15 M. Die Kationen werden beispeilsweise als Nitrate, Acetate, Sulfate oder Halogenide verwendet. Bevorzugt werden die Chloride.

Die dritte für das Verfahren der Erfindung benötigte Substanz ist ein Polyanion. Beispiele für geeignete Polyanionen sind sulfatierte Polysaccharide, wie Heparin, Dextransulfat und Mepesulfat oder Phosphowolframate. Bevorzugt wird Heparin, beispielsweise als Natriumsalz. Geeignet sind auch Polysulfate, wie Natriumpolyäthanolsulfonat und ähnliche Verbindungen. Die geeigneten Konzentrationen liegen im allgemeinen zwischen 0,005 und 0,5%.

Gemäß einer speziellen Ausführungsform der Erfindung wird außerden ein esterspaltendes Enzym zugesetzt. Beispiele für geeignete, esterspaltende Enzyme sind Lipasen, Esterasen. Besonders bevorzugt wird eine Lipase aus Rhizopus arrhizus oder eine Cholesterinestrasse. Durch den Zusatz eines derartigen esterspaltenden Enzyms wird die Genauigkeit des Verfahrens erhöht, da sich hierdurch ein durch mitgefälltes VLDL hervorgerufener Fehler vollständig eliminieren läßt. Ohne Verwendung des esterspaltenden Enzyms tritt häufig eine lag-Phase auf, so daß mit einer Messung erst nach 1 bis 2 Minuten begonnen werden kann. Dies entfällt in Gegenwart des esterspaltenden Enzyms, so daß die Bestimmungsdauer hierdurch auch abgekürzt wird. Bei dieser bevorzugten Ausführungsform der Erfindung werden daher sowohl Praktikabilität als auch Empfindlichkeit erhöht.

Die turbidimetrische Messung kann vorzugsweise kinetisch erfolgen. Von besonderem Vorteil ist dabei die Möglichkeit, die Messung kinetisch, also innerhalb eines vorbestimmten Zeitintervalls, durchzuführen.

Die Einstellung eines pH-Werts zwischen 7 und 9 erfolgt in der dem Fachmann bekannten Weise, zweckmäßig unter Verwendung eines Puffers. Prinzipiell sind die im angegebenen Bereich puffernden Substanzen verwendbar. Als geeignete Puffer können z.B. Imidazol/HCl sowie Tra/HCl genannt werden. Bevorzugt wird Tris/HCl.

Ein weiterer Gegenstand der Erfindung ist ein Reagens zur direkten turbidimetrischen bestimmung der $\beta$-Lipoproteine (LDL) in Körperflüssigkeiten, wie Serum oder Plasma, enthaltend ein Polyanion und ein Salz eines zweiwertigen Metalls, welches dadurch gekennzeichnet ist, daß es zusäßlich 1 bis 35 g pro Liter eines mehrzähligen Liganden als Komplexbildner und Puffersubstanz, pH 7 bis 9, enthält.

Hinsichtlich der einzelnen Bestandteile und ihrer Mengen gelten die oben im Zusammenhang mit dem Verfahren gemachten Ausführungen entsprechend. Vorzugsweise enthält das erfindungsgemäße Reagens daher auch zusätzlich ein esterspaltendes Enzym eine Lipase, besonders bevorzugt eine Lipase aus Rhizopus arrhizus.

Das erfindungsgemäße Reagens enthält den Komplexbildner vorzugsweise bereits in einem mit dem zweiwertigen Metall, d.h. dem Kation, komplexierten Zustand. Die bevorzugte Menge dieses Komplexsalzes beträgt 2 bis 25 g, bezogen auf die zur Herstellung von 1 l bestimmte Menge. Das Polyanion liegt zweckmäßig in einer Menge von 0,02 bis 0,5% vor. Wird das als Polyanion bevorzugte Heparin-natrium verwendet, so entspricht dies 30 000 bis 750 000 USP, wiederum bezogen auf die zur Herstellung von 1 l flüssiges Reagens vorgesehene Menge.

Hinsichtlich der Puffersubstanz gelten wiederum die obigen Auffführungen zum Verfahren. Zweckmäßig liegt die Puffersubstanz in solcher Menge vor, daß die Konzentration im flüssigen Reagens

3

O 035 211

100 bis 150 mMol/l, vorzugsweise 120 bis 140 mMol/l beträgt. Dies gilt insbesondere für den bevorzugten Tris/HCl-Puffer.

Falls ein esterspaltendes Enzym vorliegt, beträgt dessen Menge zweckmäßig 100 bis 6000 U/l gelöstes Reagens, vorzugsweise 500 bis 2000 U.

Ein bevorzugtes Reagens nach der Erfindung weist daher folgende Zusammensetzung auf:
0,02 bis 0,5 Gew.-% Heparin-natrium (30000 bis 750 000 USP/l), Dextransulfat oder Polyphosphorwolframsäure,
2 bis 25 g/l des Komplexsalzes aus Calcium und Äthylendiamintetraessigsäure,
15 bis 50 mMol/l $CaCl_2$,
100 bis 150 mMol/l Tris/HCl, pH 7 bis 9
und gegebenenfalls
500 bis 2000 U/l esterspaltendes Enzym.

Ebenso gute Ergebnisse liefert ein Reagens obiger Zusammensetzung, welches eine äquivalente Menge Dextransulfat anstelle von Heparin-natrium enthält.

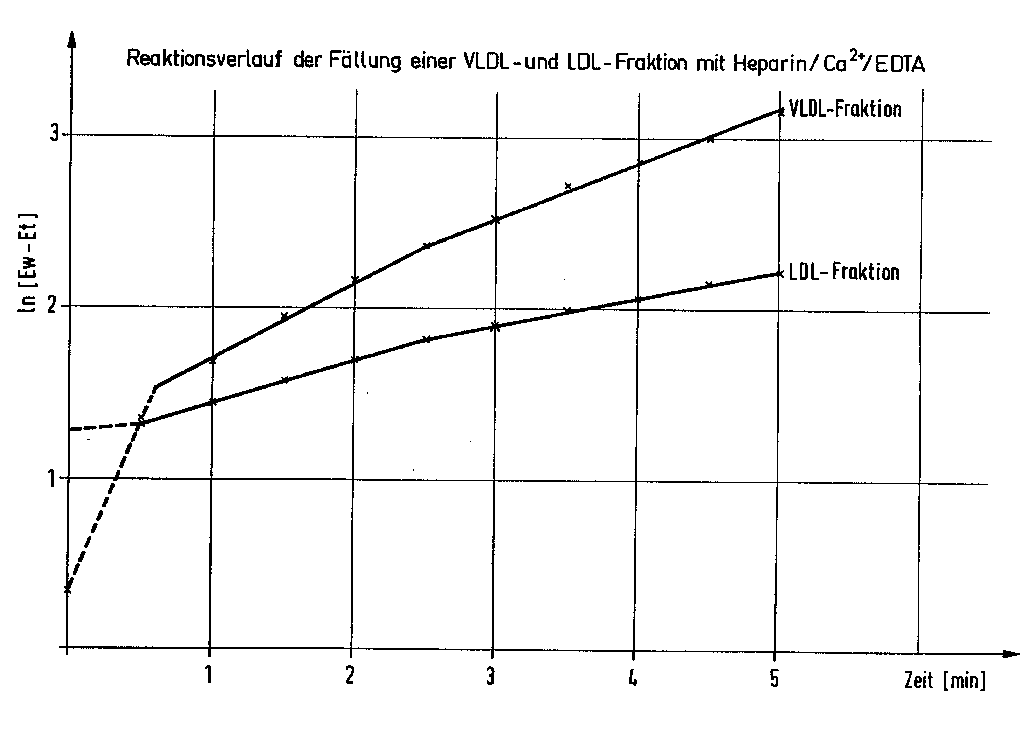

Wie oben näher erläutert, wird durch die Erfindung erreicht, daß die VLDL-Fällung innerhalb des Meßintervalls vernachlässigt werden kann (vgl. Tabelle 1). In der Figur der beigefügten Zeichnung ist die VLDL- und die LDL-Fällungsreaktion unter den Bedingungen von Beispiel 1 halblogarithmisch aufgetragen. Man erkennt, daß völlig unterschiedliche Reaktionsgeschwindigkeitskonstanten erhalten werden. Dies hat zur Folge, daß erfindungsgemäß beide Reaktionsverläufe unterschieden werden können.

Bei dem bekannten Verfahren, bei welchem mittels Zugabe von Heparin und Calcium LDL und VLDL gemeinsam ausgefällt werden, beträgt der Fehler, bezogen auf die LDL-Fraktion 30%. Erfindungsgemäß hingegen wird bereits in der Ausführungsform ohne esterspaltendes Enzym dieser Fehler auf weniger als 8% verringert.

Das erfindungsgemäße Verfahren und Reagens zeichnet sich somit dadurch aus, daß erstmals spezifisch LDL in einer einzigen, leicht durchführbaren Reaktion bestimmt werden kann. Geringe Störungen durch noch mitgefälltes VLDL können durch Zusatz von Esterase verhindert werden. Durch die bevorzugt nach dem Prinzip der kinetischen fixed-time-Methode durchgeführte Trübungsmessung ergeben sich folgende Vorteile:

1. Es entfällt eine Probenleerwertbestimmung.
2. Kinetische fixed-time-Methoden eignen sich bevorzugt zur Durchführung der bestimmung an modernen Aalysenautomaten. Dieses Verfahren ist somit leicht an die in einem modernen Routinelabor stehenden Analysengeräte adaptierbar.

Als Meßgröße dient die durch die Trübung im Meßintervall auftretende Extinktionszunahme. Diese wird vorzugsweise nach dem kinetischen Verfahren durchgeführt. Die Auswertung des Tests erfolgt über eine mittels Standardproben erstellte Eichkurve.

Ohne Probenvorverdünnung sind erfindungsgemäß LDL-Werte bis zu ca. 600 mg/dl bestimmbar.

Die folgenden Beispiele erläutern die Erfindung weiter. Der in allen beispielen eingesetzte Salzkomplex wurde nach folgendem Verfahren hergestellt:

36 g Äthylendiamintetraessigsäure werden in 400 ml bidest. Wasser gelöst. Nach Filtration werden 16 g $CaCl_2$ zu dieser Lösung gegeben. Nach 15 Minuten Rühren bei Raumtemperatur wird die Suspension einen Tag bei 4°C aufbewahrt und anschließend durch einen Glasfilter abgesaugt, chloridfrei gewaschen und getrocknet.

Beispiel 1

Die nachstehend beschriebene Bestimmung wurde nach dem kinetischen fixed-time-Verfahren unter Verwendung folgender Reagenzien durchgeführt:

Reagens 1: 124 mMol/l Tris/HCl, pH 7,5
151000 USP/l Heparin
20 g/l $Ca^{2+}$/EDTA (Salzkomplex)

Reagens 2: 0,68 Mol/l $CaCl_2$

Bestimmungsansatz:
Meßstrahlung: Hg 365 nm; Schichtdicke der Küvette: 1 cm, Meßtemperatur: 25°C.
2,5 ml des Reagens 1 werden in die Küvette pipettiert, 0,05 ml Probe zugesetzt, gemischt und mit 0,1 ml Reagens 2 die Reaktion gestartet. $E_1$ wird 90 Sekunden nach Start abgelesen, $E_2$ 300 Sekunden nach Start. Die so erhaltene Extinktionsdifferenz $\Delta E = E_2 - E_1$ wird zur Auswertung herangezogen.

Auswertung:
Die gemessene Extinktionsdifferenz wird über eine durch Einsatz einer bzw. mehrerer Standardproben hergestellte Bezugskurve zur LDL-Konzentration in Beziehung gesetzt.
Setzt man bei diesem Beispiel zum einen gereinigtes VLDL, zum anderen VLDL-freies Serum ein und wertet die bei den verschiedenen Zeiten gemessenen Extinktionen halblogarithmisch aus, so erhält

4

**O 035 211**

man die in der Zeichnung angegebenen unterschiedlichen Reaktionskinetiken für VLDL und LDL. Die unterschiedlichen Steigungen verdeutlichen dieses.

Beispiel 2 (mit Lipase-Zusatz)

Die Messung erfolgte nach dem fixed-time-Verfahren.

Reagens 1: 124 mMol/l Tris/HCl, pH 7,5
151000 USP/l Heparin
20 g/l Ca-EDTA
1500 U/l Lipase

Reagens 2: 0,68 Mol/l $CaCl_2$

Der Bestimmungsansatz wird wie in Beispiel 1 beschrieben durchgeführt. 2,5 ml des Reagens 1 in Küvette pipettieren, 0,05 ml Probe zusetzen, mischen und mit 0,1 ml Reagens 2 die Reaktion starten. $E_1$ wird 30 bis 90 Sekunden nach Start der Reaktion gemessen, $E_2$ 300 Sekunden nach Start. Die so erhaltene Extinktionsdifferenz $\Delta E = E_2 - E_1$ wird zur Auswertung herangezogen.

Zum Vergleich wurde die Bestimmung nach der oben unter 2. angegebenen bekannten Methode wiederholt. Die Ergebnisse zeigt nachstehende Tabelle 1.

TABELLE 1

Einfluß unterschiedlicher Fällungsreagenzien auf die
VLDL- bzw. LDL-Fällung

| | Heparin/$Ca^{2+}$ $\Delta E$ (90–300 Sekunden) | Heparin/$Ca^{2+}$/ EDTA, $\Delta E$ (90–300 Sekunden) | Heparin/$Ca^{2+}$/ EDTA – Lipase $\Delta E$ (90–300 Sekunden) |
|---|---|---|---|
| VLDL-Fraktion | 0,014 | 0,006 | 0,000 |
| % Fehler durch VLDL bezüglich LDL | 30 | 7 | 0 |

Beispiel 3

Es wird wie in Beispiel 2 beschrieben vorgegangen, jedoch wird ein Reagens folgender Zusammensetzung verwendet:

Reagens 1: 124 mMol/l Tris/HCl, pH 7,5
0,1% Polyphosphorwolframsäure
24 g/l Ca/EDTA
1500 U/l Lipase

Reagens 2: 0,23 Mol/l $CaCl_2$

Die Ergebnisse entsprechen denen von Beispiel 2.

**Patentansprüche**

1. Verfahren zur direkten turbidimetrischen Bestimmung der ß-Liproproteine (LDL) in Körperflüssigkeiten durch Fällung mit Polyanion und einem zweiwertigen Kation, dadurch gekennzeichnet, daß man die Fällung in Gegenwart von 1 bis 35 g pro Liter eines mehrzähligen Liganden als Komplexbildner bei einem pH-Wert zwischen 7 und 9 durchführt.

2. Verfahren nach Anspruch 1, dadurch gekennzeichnet, daß man außerdem ein esterspaltendes Enzym zusetzt.

3. Verfahren nach Anspruch 2, dadurch gekennzeichnet, daß man als esterspaltendes Enzym eine Lipase aus Rhizopus arrhizus verwendet.

4. Verfahren nach einem der vorhergehenden Ansprüche, dadurch gekenzeichnet, daß man als Komplexbildner einen sechszähligen Liganden verwendet.

5. Verfahren nach Anspruch 4, dadurch gekennzeichnet, daß man als sechszähligen Liganden Äthylendiamintetraessigsäure (EDTA) verwendet.

5

6. Verfahren nach einem der vorhergehenden Ansprüche, dadurch gekenzeichnet, daß man als Kation Erdalkali- oder Mangankationen verwendet.

7. Verfahren nach einem der vorhergehenden Ansprüche, dadurch gekenzeichnet, daß man Kation und Komplexbildner in stöchiometrischer Menge einsetzt.

8. Verfahren nach einem der vorhergehenden Ansprüche, dadurch gekenzeichnet, daß man Komplexbildner und Kation zuerst miteinander umsetzt und den gebildeten salzartigen Komplex, gegebenenfalls nach vorheriger Isolierung, der zu untersuchenden Probe zusetzt.

9. Verfahren nach einem der vorhergehenden Ansprüche, dadurch gekenzeichnet, daß man als Polyanion Heparin-Na, Polyphosphorwolframsäure oder Dextransulfat verwendet.

10. Verfahren nach einem der vorhergehenden Ansprüche, dadurch gekenzeichnet, daß man die Bestimmung kinetisch durchführt durch Messung in einem vorbestimmten Zeitintervall.

11. Verfahren nach einem der vorhergehenden Ansprüche, dadurch gekenzeichnet, daß man als Puffer Tris/HCl verwendet.

12. Reagens zur direkten turbidimetrischen Bestimmung der $\beta$-Liproproteine (LDL) in Körperflüssigkeiten enthaltend ein Polyanion und ein Salz eines zweiwertigen Metalls, dadurch gekenzeichnet, daß es zusätzlich 1 bis 35 g pro Liter eines mehrzähligen Liganden als Komplexbildner und Puffersubstanz, pH 7 bis 9, enthält.

13. Reagens nach Anspruche 13, dadurch gekenzeichnet, daß es zusätzlich ein esterspalten des Enzym enthält.

14. Reagens nach Anspruche 13, dadurch gekenzeichnet, daß das esterspaltende Enzym Lipase aus Rhizopus arrhizus ist.

15. Reagens nach Anspruche 12, dadurch gekenzeichnet, daß der Komplexbildner ein sechszähliger Ligand ist.

16. Reagens nach Anspruche 15, dadurch gekenzeichnet, daß es
0,02 bis 0,5 Gew.-% Heparin-natrium (30000 bis 750 000 USP), Dextransulfat oder Polyphosphorwolframsäure,
2 bis 35 g/l des Komplexsalzes aus Calcium$^{++}$ und Äthylendiamintetraessigsäure,
15 bis 50 mMol/l CaCl$_2$,
100 bis 150 mMol/l Tris/HCl, pH 7 bis 9
und gegebenenfalls
100 bis 6000 U/l esterspaltendes Enzym enthält.

## Revendications

1. Procédé de détérmination turbidimétrique directe des $\beta$-lipoprotéines (LDL) dans les liquides corporels par précipitation avec un polyanion et un cation bivalent, caractérisé en ce qu'on effectue la précipitation en présence de 1 à 35 g par litre d'un ligand polycoordinateur comme complexant à une valeur de H entre 7 et 9.

2. Procédé suivant la revendication 1, caractérisé en ce qu'on ajoute en outre une enzyme scindant les esters.

3. Procédé suivant la revendication 2, caractérisé en ce qu'on utilise comme enzyme scindant les esters une lipase de Rhizopus arrhizus.

4. Procédé suivant l'une des revendications précédentes, caractérisé en ce qu'on utilise comme complexant un ligand hexacoordinateur.

5. Procédé suivant la revendication 4, caractérisé en ce qu'on utilise comme ligand hexacoordinateur l'acide éthylènediaminotétraacétique (EDTA).

6. Procédé suivant l'une des revendications précédentes, caractérisé en ce qu'on utilise comme cation des cations alcalino-terreux ou de manganèse.

7. Procédé suivant l'une des revendications précédentes, caractérisé en ce qu'on utilise le cation et le complexant en quantités stoechiométriques.

8. Procédé suivant l'une des revendications précédentes, caractérisé en ce qu'on fait réagir d'abord entre eux le complexant et le cation et en ce qu'on ajoute le complexe salin formé, le cas échéant après isolement préalable, à l'échantillon à étudier.

9. Procédé suivant l'une des revendications précédentes, caractérisé en ce qu'on utilise comme polyanion de l'héparine-Na, de l'acide polyphosphotungstique ou du sulfate de dextrane.

10. Procédé suivant l'une des revendications précédentes, caractérisé en ce qu'on effectue la détermination cinétiquement par mesure dans un intervalle de temps déterminé à l'avance.

11. Procédé suivant l'une des revendications précédentes, caractérisé en ce qu'on utilise comme tampon du tris/HCl.

12. Réactif pour la détermination turbidimétrique directe des $\beta$-lipoprotéines (LDL) dans des liquides corporels, contenant un polyanion et un sel d'un métal bivalent, caractérisé en ce qu'il contient en outre 1 à 35 g par litre d'un ligand polycoordinateur comme complexant et une substance tampon, pH 7 à 9.

13. Réactif suivant la revendication 12, caractérisé en ce qu'il contient en outre une enzyme scindant l'ester.

**0 035 211**

14. Réactif suivant la revendication 13, caractérisé en ce que l'enzyme scindant l'ester est la lipase de Rhizopus arrhizus.

15. Réactif suivant la revendication 12, caractérisé en ce que le complexant est un ligand hexacoordinateur.

16. Réactif suivant la revendication 15, caractérisé en ce qu'il contient:

0,02 à 0,5% en poids d'héparine-sodium (30.000 à 750.000 USP), du sulfate de dextrane ou de l'acide polyphosphotungstique,

2 à 35 g/l du sel complexe de calcium⁺⁺ et de l'acide éthylènediaminotétracétique,

15 à 50 mmole/l de CaCl₂,

100 à 150 mmole/l de tris/HCl, pH 7 à 9,

et le cas échéant

100 à 6000 U/l d'enzyme scindant l'ester.

**Claims**

1. Process for the direct turbidimetric determination of the β-lipoproteins (LDL) in body fluids by precipitation with a polyanion and a divalent cation, characterised in that one carries out the precipitation in the presence of 1 to 35 g. per litre of a polyligand as complex former at a pH value between 7 and 9.

2. Process according to claim 1, characterised in that one also adds an ester-splitting enzyme.

3. Process according to claim 2, characterised in that one uses a lipase from *Rhizopus arrhizus* as ester-splitting enzyme.

4. Process according to one of the preceding claims, characterised in that one uses a hexaligand as complex former.

5. Process according to claim 4, characterised in that one uses ethylenediamine-tetraacetic acid (EDTA) as hexaligand.

6. Process according to one of the preceding claims, characterised in that one uses alkali earth metal or manganese cations as cation.

7. Process according to one of the preceding claims, characterised in that one uses cation and complex former in stoichiometric amount.

8. Process according to one of the preceding claims, characterised in that one first reacts complex former and the cation with one another and adds the sample to be investigated to the salt-like complex formed, possibly after previous isolation.

9. Process according to one of the preceding claims, characterised in that one uses heparin-Na, polyphosphotungstic acid or dextran sulphate as polyanion.

10. Process according to one of the preceding claims, characterised in that one carries out the determination kinetically by measurement in a predetermined time interval.

11. Process according to one of the preceding claims, characterised in that one uses tris/HCl as buffer.

12. Reagent for the direct turbidimetric determination of the β-lipoproteins (LDL) in body fluids, containing a polyanion and a salt of a divalent metal, characterised in that it additionally contains 1 to 35 g. per litre of a polyligand as complex former and buffer substance, pH 7 to 9.

13. Reagent according to claim 12, characterised in that it additionally contains an ester-splitting enzyme.

14. Reagent according to claim 13, characterised in that the ester-splitting enzyme is lipase from *Rhizopus arrhizus.*

15. Reagent according to claim 12, characterised in that the complex former is a hexaligand.

16. Reagent according to claim 15, characterised in that it contains:

0.02 to 0.5 wt.% heparin sodium (30,000 to 750,000 USP), dextran sulphate or polyphosphotungstic acid,

2 to 35 g./l. of the complex salt of calcium⁺⁺ and ethylenediamine-tetraacetic acid,

15 to 50 mMol/l. CaCl₂,

100 to 150 mMol/l. tris/HCl, pH 7 to 9

and possibly

100 to 6000 U/l. of ester-splitting enzyme.

7

Reaktionsverlauf der Fällung einer VLDL-und LDL-Fraktion mit Heparin/Ca²⁺/EDTA